# EUROPÄISCHE PATENTANMELDUNG

(11) **EP 1 146 336 A2**
(43) Veröffentlichungstag der Anmeldung: **17.10.2001**
(21) Anmeldenummer: 01250124.3
(22) Anmeldetag: 06.04.2001
(51) Int. Cl.: G01N 33/00

(54) **Gassensoranordnung**

(30) Priorität: 13.04.2000 DE 10019853
(71) Anmelder: MSA Auer GmbH, 12059 Berlin (DE)
(72) Erfinder: Schröder, Willi, 06846 Dessau (DE)
(74) Vertreter: Wablat, Wolfgang, Dr.Dr.

(57) **Zusammenfassung**

Die Erfindung betrifft eine Gassensoranordnung, bei der ein oder mehrere Gassensoren (4) zur Überwachung oder Messung von Gas-, Dampfkonzentrationen oder Gemischen in Räumen angeordnet sind und den Gassensoren (4) ein Diffusionskörper (5) vorgeschaltet ist, ihre Ansprechempfindlichkeit durch bauliche Maßnahmen oder Verschmutzung beeinträchtigt ist und/oder die Gasatmosphäre ruhend ist. Der Sensor (4) oder die Sensoren (4.1 bis 4.n) sind in, an, über oder vor einem im Raum befindlichen Strömungskanal (1) und somit in dessen Einzugsbereich angeordnet, wobei im Strömungskanal (1) während der Messung oder Überwachung eine Strömung ausgebildet ist. Um diese auszubilden, kann der Strömungskanal mittels einer Heizung (6) künstlich erwärmt werden.

## Beschreibung

Die Erfindung ist insbesondere für Gassensoren geeignet, denen eine Diffusionsbarriere vorgeschaltet ist, deren Ansprechempfindlichkeit durch andere bauliche Maßnahmen oder Verschmutzung beeinträchtigt wird oder die bevorzugt in ruhenden Gasatmosphären eingesetzt werden.

Es ist bekannt, das bei Gasen oder Dämpfen, die über poröse Körper per Diffusion zum Sensor oder zu den Sensoren gelangen, sich Diffusionsbarrieren mit einem Diffusionsgradienten ausbilden, die den Gaswechsel beeinträchtigen, da die Gasdiffusion durch derartige Barrieren durch die Partialdruckdifferenz bestimmt wird.
Kleine Meßbereiche und geforderte hohe Ansprechempfindlichkeit sind so mit herkömmlichen Gassensoranordnungen, insbesondere unter den Bedingungen vorhandener Diffusionsbarrieren und in ruhenden Gasatmosphären, nicht mehr zu erreichen.

Aufgabe der Erfindung ist es, die dynamischen Eigenschaften der Gase und Dämpfe unter den genannten Bedingungen zu verbessern. Gelöst wird diese Aufgabe mit den kennzeichnenden Merkmalen des Anspruchs 1, vorteilhafte Ausgestaltungen sind Gegenstand der Unteransprüche. Eine andere Verwendung beschreibt Anspruch 8.

Die erfindungsgemäße Gassensoranordnung, bei der ein oder mehrere Gassensoren zur Überwachung oder Messung von Gas-, Dampfkonzentrationen oder Gemischen in Räumen angeordnet sind und den Gassensoren eine Diffusionsbarriere vorgeschaltet ist, ihre Ansprechempfindlichkeit durch bauliche Maßnahmen oder Verschmutzung beeinträchtigt ist und/oder die Gasatmosphäre ruhend ist, sieht vor, dass der Sensor oder die Sensoren in, an, über oder vor einem im Raum befindlichen Strömungskanal und somit in dessen Einzugsbereich angeordnet sind und im Strömungskanal während der Messung oder Überwachung eine Strömung ausgebildet ist. Dabei weist der Strömungskanal bevorzugt von unten nach oben.
Zum Erzeugen der Strömung kann in vorteilhafter Weise die natürliche Temperaturdifferenz genutzt werden und/oder der Strömungskanal wird mittels einer Heizung künstlich erwärmt.

Die Erfindung nutzt dabei die durch praktische Messungen gewonnene Erkenntnis aus, dass an Fernmessköpfen, welche im Diffusionsbetrieb arbeiten, die Signaländerung auch bei geringen Meßgaskonzentrationen bei der Änderung der Strömung von nahe Null zu einer geringfügigen Strömung deutlich höher ist, als bei einer Änderung von einer kleinen zu einer hohen Strömung.

In einer bevorzugten Ausführung ist der Strömungskanal als Rohr ausgebildet, das mindestens eine seitliche Öffnung, einfacherweise eine Bohrung, aufweist, über die der Gaskontakt zum Sensor besteht.

Diese Baugruppe läßt sich gut in ein Gehäuse, in dem Mess- und Auswertetechnik angeordnet ist, integrieren. Das obere und untere Rohrende durchdringen dabei die Gehäusewände. Dem Sensor kann dabei problemlos auch ein Diffusionskörper vorgeschaltet sein. Darüber hinaus bietet diese Anordnung auch die Möglichkeit, zur künstlichen Erwärmung des Strömungskanals die Verlustleistung der innerhalb des Gehäuses vorhandenen elektronischen oder elektrischen Bauelemente zu nutzen.

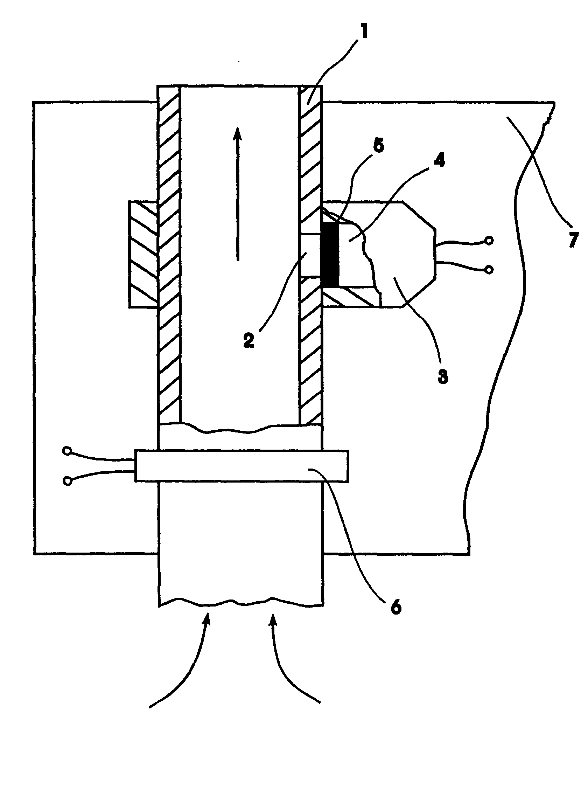

Ein Ausführungsbeispiel der Gassensoranordnung, die besonders als Brandgasmelder in Kraftwerken geeignet ist, wird anhand der Zeichnung erläutert:

Der Strömungskanal 1 ist als Rohr ausgebildet und in einem Gehäuse 7 mit Mess- und Auswertetechnik angeordnet. Die Rohrenden durchdringen die obere und untere Gehäusewand, so dass Gase oder Dämpfe ungehindert das Rohr durchströmen können. Das Rohr weist eine seitliche Öffnung 2, vorzugsweise eine Bohrung, auf, über die der Gaskontakt zum Sensor 4 besteht. Dem Sensor 4 ist ein Diffusionskörper 5 vorgeschaltet, der vom Gas/Dampf zur Messung/Überwachung durchdrungen werden muss. Zur künstlichen Erwärmung des als Strömungskanal 1 dienen Rohres mittels der Heizung 6 wird die Verlustleistung der elektronischen oder elektrischen Bauelementen innerhalb des Gehäuses 7 genutzt, was bei diesen gleichzeitig einen Kühleffekt bewirkt. Gleichzeitig wird so während der Messung oder Überwachung eine ständige Strömung im Rohr ausgebildet, die die Gasdiffusion und damit den Gas- oder Dampfwechsel am Sensor 4 beschleunigt. Die Ansprechempfindlichkeit des Sensors 4 wird so deutlich gesteigert.

Die erfindungsgemäße Sensoranordnung ist auch auf Messungen und Überwachungen im Wasser übertragbar, wo sich z.B. durch Bläschenbildung an Wasserelektroden die Ansprechemfindlichkeiten verändern. Anstelle des Strömungskanals tritt hier ein Tauchrohr und anstelle des Gassensors beispielsweise eine Wasserelektrode. Durch das Erzeugen einer ständigen Strömung im Tauchrohr, u.a. mittels einer künstlichen Beheizung, wird bei der Wasserelektrode, die sich im Strömungseinzugsbereich des Tauchrohres befindet, die Bläschenablagerung verhindert oder vermindert, so dass deren Ansprechempfindlichkeit deutlich steigt.

### Bezugszeichenliste

- 1: Strömungskanal
- 2: Öffnung
- 3: Sensorgehäuse
- 4: Sensor
- 5: Diffusionskörper
- 6: Heizung
- 7: Gehäuse

## Patentansprüche

1. Gassensoranordnung, bei der ein oder mehrere Gassensoren zur Überwachung oder Messung von Gas-, Dampfkonzentrationen oder Gemischen in Räumen angeordnet sind und den Gassensoren eine Diffusionsbarriere vorgeschaltet ist, ihre Ansprechempfindlichkeit durch bauliche Maßnahmen oder Verschmutzung beeinträchtigt ist und/oder die Gasatmosphäre ruhend ist, **dadurch gekennzeichnet, dass**
der Sensor (4) oder die Sensoren (4.1 bis 4.n) in, an, über oder vor einem im Raum befindlichen Strömungskanal (1) und somit in dessen Einzugsbereich angeordnet sind und im Strömungskanal (1) während der Messung oder Überwachung eine Strömung ausgebildet ist.

2. Gassensoranordnung nach Anspruch 1, **dadurch gekennzeichnet, dass**
der Strömungskanal (1) von unten nach oben weist:

3. Gassensoranordnung nach Anspruch 1, **dadurch gekennzeichnet, dass**
der Strömungskanal mittels einer Heizung (6) künstlich erwärmt ist.

4. Gassensoranordnung nach Anspruch 1, **dadurch gekennzeichnet, dass**
der Strömungskanal (1) als Rohr ausgebildet ist, das mindestens eine seitliche Öffnung (2), vorzugsweise eine Bohrung, aufweist, über die der Gaskontakt zum Sensor (4) besteht.

5. Gassensoranordnung nach Anspruch 1, **dadurch gekennzeichnet, dass**
der Strömungskanal (1) ein Gehäuse (7), in dem Mess- und Auswertetechnik angeordnet ist, durchdringt, dabei unten und oben offen ist, sich der Sensor (4) innerhalb des Gehäuses (7) befindet und über eine Öffnung (2) mit dem inneren des Strömungskanals (1) verbunden ist.

6. Gassensoranordnung nach Anspruch 4 oder 5, **dadurch gekennzeichnet, dass**
dem Sensor (4) ein Diffusionskörper (5) vorgeschaltet ist, der vom Gas/Dampf zur Messung/Überwachung durchdrungen werden muss.

7. Gassensoranordnung nach Anspruch 3 und 6, **dadurch gekennzeichnet, dass**
zur künstlichen Erwärmung des Strömungskanals (1) die Verlustleistung von elektronischen oder elektrischen Bauelementen innerhalb des Gehäuses (7) genutzt wird.

8. Verwendung der Gassensoranordnung nach einem der Ansprüche 1 bis 7 zu Mess- und Überwachungszwecken im Wasser, wobei der Strömungskanal als Tauchrohr ausgebildet ist und der oder die Sensoren Wasserelektroden sind.
